# EUROPEAN PATENT SPECIFICATION

(11) **EP 2 160 478 B1**
(45) Date of publication and mention of the grant of the patent: **27.08.2014**
(21) Application number: 08770088.6
(22) Date of filing: 04.06.2008
(51) Int. Cl.: C12Q 1/68, G01N 33/50

(54) **PREDICTIVE DIAGNOSTICS FOR KIDNEY DISEASE**
PROGNOSTISCHE DIAGNOSE VON NIERENERKRANKUNGEN
DIAGNOSTICS PRÉDICTIFS POUR MALADIE RÉNALE

(30) Priority: 06.06.2007 US 942294 P
(43) Date of publication of application: 10.03.2010
(73) Proprietor: Siemens Healthcare Diagnostics Inc., Tarrytown, NY 10591 (US)
(72) Inventor: PUGIA, Michael, Granger, IN 46530 (US)
(74) Representative: Auerbach, Bernhard Konrad
(86) International application number: PCT/US2008/065717
(87) International publication number: WO 2008/154238

(56) References cited:
- US-A1- 2001 055 816
- D'AMICO GIUSEPPE ET AL: "Urinary protein and enzyme excretion as markers of tubular damage" CURRENT OPINION IN NEPHROLOGY & HYPERTENSION, LIPPINCOTT WILLIAMS & WILKINS, LTD, GB, vol. 12, no. 6, 1 November 2003 (2003-11-01), pages 639-643, XP008123969 ISSN: 1062-4821
- PUGIA MICHAEL J ET AL: "Pathophysiology and diagnostic value of urinary trypsin inhibitors" CLINICAL CHEMISTRY AND LABORATORY MEDICINE, vol. 43, no. 1, 2005, pages 1-16, XP008124055 ISSN: 1434-6621
- HAN W K ET AL: "BIOLOGIC MARKERS FOR THE EARLY DETECTION OF ACUTE KIDNEY INJURY" CURRENT OPINION IN CRITICAL CARE, CURRENT SCIENCE, PHILADELPHIA, PA, US LNKD- DOI:10.1097/01.CCX.0000145095.90327.F2, vol. 10, no. 6, 1 January 2004 (2004-01-01), pages 476-482, XP008062501 ISSN: 1070-5295
- HORCAJADA JUAN P ET AL: "Evaluation of inflammatory and renal-injury markers in women treated with antibiotics for acute pyelonephritis caused by Escherichia coli." CLINICAL AND DIAGNOSTIC LABORATORY IMMUNOLOGY, vol. 11, no. 1, January 2004 (2004-01), pages 142-146, XP002590465 ISSN: 1071-412X
- SCHERBERICH J E: "Renale Gewbsproteinurie bei interstitiellen und toxisch bedingten Nierenerkrankungen" NIEREN- UND HOCHDRUCKKRANKHEITEN, DUSTRI VERLAG, DE, vol. 31, no. 10, 1 October 2002 (2002-10-01), pages 463-470, XP008123967 ISSN: 0300-5224
- GORDJANI NADER ET AL: "Urinary excretion of adenosine deaminase binding protein in neonates treated with tobramycin" PEDIATRIC NEPHROLOGY, SPRINGER VERLAG, BERLIN, DE LNKD- DOI:10.1007/BF00866714, vol. 9, no. 4, 1 August 1995 (1995-08-01), pages 419-422, XP008123978 ISSN: 0931-041X [retrieved on 2004-12-29]
- M.W. WOLF AND J. BOLDT: "kidney-specific proteins: markers for detection of renal dysfunction after cardiac surgery" CLIN RES CARIOL, vol. 2, no. S1, January 2007 (2007-01), pages S103-S107, XP002590467
- TOKI N ET AL: "URINARY TRYPSIN INHIBITOR AND URO KINASE ACTIVITIES IN RENAL DISEASES" ACTA HAEMATOLOGICA (BASEL), vol. 67, no. 2, 1982, pages 109-113, XP008124047 ISSN: 0001-5792
- THOMPSON R E ET AL: "ADENOSINE DEAMINASE BINDING PROTEIN, A NEW DIAGNOSTIC MARKER FOR KIDNEY DISEASE" CLINICAL CHEMISTRY, AMERICAN ASSOCIATION FOR CLINICAL CHEMISTRY, WASHINGTON, DC, vol. 31, no. 5, 1 January 1985 (1985-01-01), pages 679-683, XP002909654 ISSN: 0009-9147
- MCBRIDE WILLIAM T ET AL: "Methylprednisolone favourably alters plasma and urinary cytokine homeostasis and subclinical renal injury at cardiac surgery" CYTOKINE, vol. 27, no. 2-3, 21 July 2004 (2004-07-21), pages 81-89, XP002590466 ISSN: 1043-4666
- SUZUKI ET AL: "EXCRETION OF BIKUNIN AND ITS FRAGMENTS IN THE URINE OF PATIENTS WITH RENAL STONES" JOURNAL OF UROLOGY, LIPPINCOTT WILLIAMS & WILKINS, BALTIMORE, MD, US LNKD- DOI:10.1016/S0022-5347(05)66143-5, vol. 166, no. 1, 1 July 2001 (2001-07-01), pages 268-274, XP005545416 ISSN: 0022-5347
- EMEIGH HART S G: "Assessment of renal injury in vivo" JOURNAL OF PHARMACOLOGICAL AND TOXICOLOGICAL METHODS, ELSEVIER, NEW YORK, NY, US LNKD- DOI:10.1016/J.VASCN.2005.04.006, vol. 52, no. 1, 1 July 2005 (2005-07-01), pages 30-45, XP004985391 ISSN: 1056-8719
- THOMPSON ET AL.: 'Adenosine Deaminase Binding Protein, a New Diagnostic Marker for Kidney Disease' CLINICAL CHEMISTRY vol. 31, no. 5, May 1985, pages 679 - 683, XP002909654

## Description

### FIELD

The present invention relates to markers for kidney disease.

### BACKGROUND OF THE INVENTION

Urinalysis strips have long used protein measurements for diagnosing glomerular nephritis and diabetic glomerulerosis. These inflammatory conditions are the leading cause of end stage renal disease. Protein is spilled from the kidney after damage to the cells has occurred. Recently microalbuminuria and creatinine ratios have been added to urinalysis strips to allow detection of smaller amounts of damage earlier in the disease process. The measurement of albumin in urine reflects damage to the basement membrane in the glomerulus of the kidney. Increased damage leads to higher glomerular filtration rates and can progress to renal failure. Increased blood pressure during a pro-inflammatory response, however, can also cause more albumin to be spilled into urine. Accordingly, albuminuria is also a measure of vascular permeability and is an independent risk factor for cardiovascular disease. Because albuminuria is an indirect measure of inflammation and does not directly play a part in the inflammatory response, the measurement of albuminuria can over estimate renal damage in the presence of hypertension or systemic inflammation. Serial measurements of albuminuria every three months are recommended in order to assure that measurement accurately reflects kidney damage. There is a need for kidney disease markers with greater specificity. The present invention addresses these and other needs.

### SUMMARY

The present invention provides, *inter alia,* methods for identifying whether a subject has kidney disease or is at an increased risk for developing kidney disease.

Methods of the present invention comprise the steps of determining the level of bikunin and the level of the kidney cell death marker ADBP-26 in a biological sample obtained from a subject and determining whether a subject has kidney disease or is at an increased risk for developing kidney disease based on the levels of bikunin and the kidney cell death marker ADBP-26 in the biological sample.

The ratio of the levels of the kidney cell death marker ADBP-26 to the levels of bikunin in the biological sample obtained from the subject will be determined and the numerical value of the ratio will be indicative of whether the subject has kidney disease or is at an increased risk for developing kidney disease.

Methods for measuring the course of kidney disease are also provided. These methods comprise the steps of determining, at a first time point, the level of bikunin and the level of the kidney cell death marker ADBP-26 in a biological sample obtained from the subject; determining at a second time point, the level of bikunin and the level of a kidney cell death marker in a biological sample obtained from the subject; and comparing the first measurement and the second measurement; wherein the comparative measurements determine the course of kidney disease. The ratio of the kidney cell death marker ADBP-26 to bikunin in a biological sample obtained from the subject will be determined.

In certain embodiments, the methods of the present invention will identify whether a subject has bikunin induced inflammatory kidney cell death.

In certain embodiments, the methods of the present invention will identify whether a subject has non-inflammatory cell death.

In certain embodiments, the methods of the present invention will identify whether a subject has bikunin induced inflammation without cell death.

Also provided are kits comprising a means for measuring adenosine deaminase binding protein (ADBP-26) and bikunin in a biological sample; and optionally instructions for use and interpretation of the kit results.

### BRIEF DESCRIPTION OF THE DRAWINGS

Figure 1: Bikunin - Bikunin contains one O-linked glycoside and one N-linked glycoside chain connected to the inhibitory Kunitz 1 domain on the N-terminus (uristatin-2).
Figure 2: Formation of bikunin and uristatin. In the top section of the figure, bikunin is linked to H1 and H2 heavy chains or just an H3 chain; the two forms are found primarily in plasma. In the bottom section of the figure, serine protease splits bikunin. As bikunin fragments further into uristatin, the amount in urine increases over the amount found in blood.
Figure 3: A graph showing kidney cell death and ADBP-26 release upon exposure of kidney cells to bikunin.

### DETAILED DESCRIPTION OF ILLUSTRATIVE EMBODIMENTS

### I. Introduction

The present inventors have discovered that bikunin induces kidney cell death and further, in order to appropriately identify subjects that have kidney disease or are at an increased risk of developing kidney disease, levels of both bikunin and the kidney cell death marker ADBP-26 should be measured. By measuring levels of both bikunin and ADBP-26, the inventors have found that it is possible to group subjects into those having bikunin induced inflammatory cell death, non-inflammatory cell death, or bikunin induced inflammation without cell death. Treatment regimens can then be tailored to the subject depending on the cause of the inflammation and/or cell death.

It is to be understood that the invention described herein is not limited to particular methods, reagents, compounds, compositions or biological systems, which can, of course, vary. It is also to be understood that the terminology used herein is for the purpose of describing particular embodiments only, and is not intended to be limiting. As used in this specification and the appended claims, the singular forms "a", "an" and "the" include plural referents unless the content clearly dictates otherwise.

The term "about" as used herein when referring to a measurable value such as an amount, a temporal duration, and the like, is meant to encompass variations of ±20% or ±10%, more preferably ±5%, even more preferably ±1%, and still more preferably ±0.1% from the specified value, as such variations are appropriate to perform the disclosed methods.

The invention employs biological fluids as the biological samples for use in the present invention. Preferably, the biological fluid is urine or blood, but other biological fluids can be used. Methods of obtaining biological samples from subjects are known in the art and are not described herein in detail.

Except when noted, the terms "subject" or "patient" are used interchangeably and refer to mammals such as human patients and non-human primates, as well as experimental animals such as rabbits, rats, and mice, and other animals.

### II. Bikunin ("Bik")

Bikunin, also called bik, is one of the primary inhibitors of the trypsin family of serine proteases. In particular, bikunin suppresses serine proteases during inflammation. Much of the bikunin present in the circulation is bound to the proinhibitors (IαI and PαI). During inflammation, degradation of the IαI by proteases occurs and bikunin is freed from the complex and passes into urine. The concentration of bikunin in urine is less than 7.5 mg/L in 99% of an apparently healthy population and about 7.5 mg/L to about 200 mg/L in those with infections. Because bikunin inhibits the action of proteases that are present in inflammatory disorders, it is considered to be a part of innate immunocompetency. Inhibition of inflammatory serine protease generally occurs in a range from about 0.03 to 800 µM.

Bikunin is known by many different names including, urinary trypsin inhibitor (uTi), mingin, human inhibitor 30 (HI-30), urinastatin, ulinastatin, and Miraclid. Molecular weights ranging from 3 to 70 kDa have been observed for Bikunin. These different molecular weight are a result of different fragmentation and glycoconjugation patterns.

Bikunin refers to a protein consisting of one or two proteinase inhibitor domains of the kunitz type that can be connected by a short peptide chain and extended by N- and C-terminated polypeptide chains. The protein is linked to a sulphated chondroitin chain and to an oligosaccharide and has a molecular weight of about 33 kDa, with a range of from about 21 to about 46 kDa.

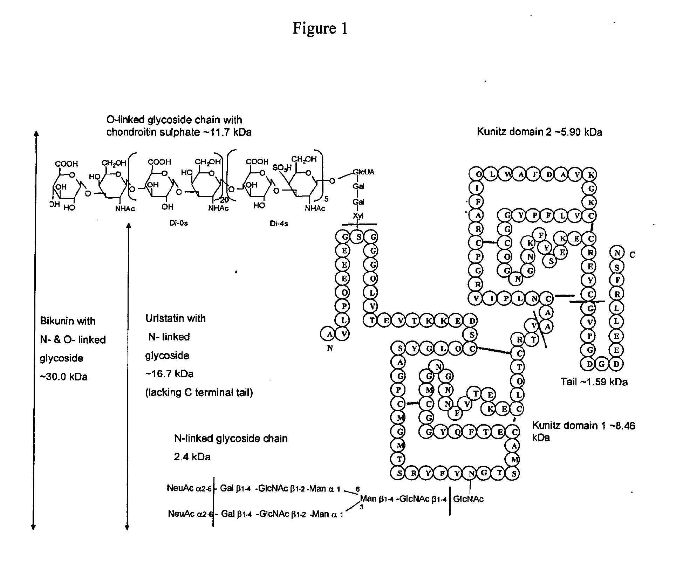

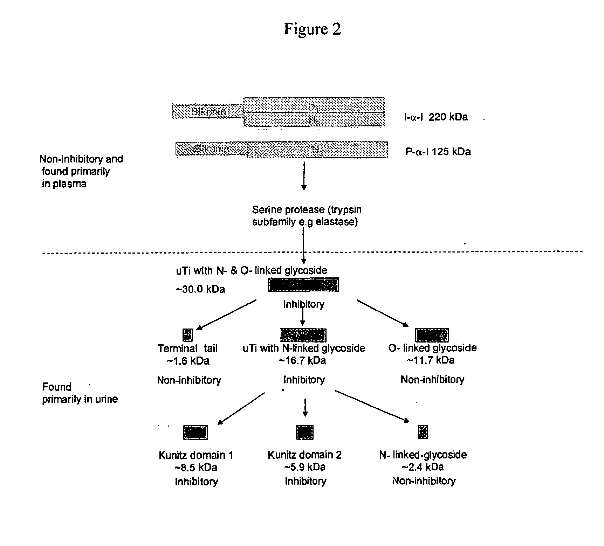

As used herein, the term bikunin includes both non-fragmented and fragmented bikunin as well as different forms of glycoconjugated bikunin and glycoconjugated fragmented bikunin, including, for example, α-1-microglubulin/bikunin precursor protein (AMBK), uristatin, uristatin-1, and uristatin-2. The glycoside chain and the kunitz domains of bikunin are provided in Figure 1. During inflammation, several of the key fragments are found in urine as provided in Figure 2.

Cleavage of the chondoroitin sulfate chain from bikunin produces uristatin. Uristatin has a monomeric molecular weight of about 17 kDa with a range of from about 11 to about 22 kDa for fragments and variants. The presence of inflammation leads to inter-α-inhibitors breakdown and forms bikunin and uristatin.

Uristatin-1 and -2 refers to protein fragments of uristatin which contain either of the kunitz type inhibitor domains 1 or 2. Uristatin-1 has a monomer molecular weight of about 6 kDa with a range of from about 2 to about 9 kDa for fragments and variants. Uristatin-2 has a monomer molecular weight of about 8.5 kDa with a range of from about 2 to about 12 kDa for fragments and variants.

AMBK refers to the protein precursor of pro-inhibitors which has three connected proteins namely the inhibitor fraction (bikunin), α-1-microglobulin, and a 25 kDA unidentified protein. It is considered to be within the definition of bikunin for purposes of the invention, without regard to its function in the biosynthesis of inhibitory serine proteases.

When measuring bikunin for the methods of the present invention, one or more of the different forms of bikunin are measured together.

### III. Kidney cell death marker

As used herein, the term "kidney cell death marker" refers to any marker that is released from kidney tissue into the urine as a result of kidney cell death. In certain embodiments, the kidney cell death will be caused by exposure of the kidney tissue to bikunin. Kidney death cell markers are highly expressed in either kidney tissue or cells and do not have significant blood concentration excreted in urine due to diseases that are not associated with kidney cell death. Kidney cell death markers can be identified by proteomic and expression profiling of kidney cells under normal and bikunin stress periods.

Kidney cell death markers include proteins (e.g., enzymes) found in the cells and tissue of the glomerular and proximal tubule. These proteins are typically bound to the cell membranes, bound to the brush border membrane, or contained within intra cellular spaces such as in the cytoplasm or lysosomes. The bound and contained markers are released because the membranes are disrupted or lysed during cell death.

Kidney cell death markers that are bound to the cell membranes include transmembrane protein of the kidney such as adenosine deaminase binding protein (ADBP-26), cation transporter exchanger (NHE3), and kidney injury molecule-1 (KIM-1).

Cell death markers of the brush border include alanine-aminopeptidase, neutral endopeptidase, and alkaline phosphatase.

Cell death markers that are intracellular proteins of the kidney include glytamyltransferase, N-acetyl-beta-D-glucosaminidase lysozyme, neutrophil gelatinase associated lipocalin (NGAL) and L-type fatty acid binding protein (L-FABP).

A large number of proteins can be found in the urine (Han and Bonventre, Current Opinion in Critical Care, 2004 10:476-842; Cheyron et al., American Journal ofKidney Disease, 2003 42(3):497-506; Jung et al., Clinical Chemistry 1986 32(10):1807-1811; Pang et al., Journal of Proteome Research, 2002 1:161-169; Trof et al., Shock 2006, 26(3):245-253; Scherberich, J.E., Renale Gewebsproteinurie bei interstitiellen und toxisch bedingten Nierenerkrankungen. Nieren- und Hochdruckkrankheiten 2002, 31 (10): 463-470). Any protein that is released from kidney tissue into the urine as a result of kidney cell death can be used as a kidney cell death marker. Kidney cell death markers are specific for kidney cell death. For example, kidney cell death markers are preferably greater than about 90% specific or even greater than about 95% specific for kidney cell death. Methods for determining specificity are well known in the art. For example, specificity of a biomarker for a disease state can determined by the equation TN/(TN+FP) wherein TN represents a true positive patient (a patient with the condition and detected as having the condition by the method use) and FP represents a false positive patient (a patient without the condition who was detected as having the condition by the method used).

### A. Membrane bound kidney cell proteins

The most widely used example of a kidney cell marker is adenosine deaminase binding protein ("ADBP-26"). This membrane bound kidney cell protein is a 120,000 dalton surface glycoprotein found on the brush border of the kidney proximal tubular epithelial cell. It is also known as Type-2 transmembrane glycoprotein ecto-Peptidase, DPPIV dipeptidyl peptidase, ADBP-26 adenosine deaminase binding protein. This trans-membrane glycoprotein exhibits serine dipeptidyl peptidase activity (DPPIV) and participates in the final stages of protein reabsorption in the kidney by hydrolysis of proline containing peptides. Therefore it is expressed in normal cell function.

ADBP-26 is also expressed as CD-26 on T cell lymphocytes during the adaptive immune response and is also up-regulated during cancer cell invasion and angiogenesis. While found in T cell lymphocytes in the blood, the high molecular weight of assures that release into urine is primarily due to kidney cell death in the proximal tubular and not from pre-renal sources. The test is >95% specific for kidney cell death. When proximal tubular cell membranes are disrupted upon death, this protein is freed from the membrane and spills into the urine. ADBP-26 has therefore been used as a marker for acute renal injury (Thompson et al., Toxicol. Pathol., Vol. 14, p. 232-237 (1986) and Thompson, R.E., Adenosine deaminase binding protein, a new diagnostic marker for kidney disease. Clin. Chem 1985, 31/5: 679-683). For example, ADBP-26 is released in high concentrations in the urine in patients with acute renal tubular necrosis (Goren, et al., Am J. Clin. Pathol., Vol. 86, p. 780-783 (1986)) as well as in patients with renal transplant rejection as well as in neonates treated with tobramycin (Gordjani, N., Urinary excretion of adenosine deaminase binding protein in neonates treated with tobramycin. Pediatr. Nephrol 1995, 9: 419-422).

As used herein, the term ADBP-26 includes both non-fragmented and fragmented ADBP-26 as well as different forms of glycoconjugated ADBP-26 and glycoconjugated fragmented ADBP-26. The sequence and glycosylation patterns of ADBP-26 can be found in Aertgeerts et al., Protein Sci. 2004 Jan; 13(1):145-54. The predicted monomer of ADBP-26 is about 110 kDa. Observed soluble forms bands can be seen at, for example, about 110 kDa, about 77kDa, about 50kDa and about 45kDa.

There are many other examples of kidney cell death markers that are cell membrane proteins that are released into the urine upon cell death. One example is the cation transporter exchanger (Na+/H+ exchanger isoform 3 or NHE3), the most abundant apical sodium transporter in the renal tubule. NHE3 has been shown to be increased in the urine of patients presenting with acute renal failure (ARF) with severe tubular cell damage (du Cheyron D Am J Kidney Dis. 2003 Sep;42(3):497-506). Another example is Kidney Injury Molecule-1 (KIM-1), a type 1 transmembrane protein whose expression is markedly up-regulated in the proximal tubule. The ectodomain of KIM-1 is shed from cell during ischemic events causing cell death (Han WK Curr Opin Crit Care. 2004, 476-82).

The cell membrane proteins are high molecular weight proteins that are highly expressed in the kidney due to a functional requirement of the kidney. Because the proteins are high molecular weight proteins, they are less likely to be in the urine due to non-renal sources.

### B. Brush border membrane proteins

Proteins located in the brush border membranes of the proximal tubule can also be used as kidney cell death markers. Exemplary proteins include alanine-aminopeptidase, neutral endopeptidase, and alkaline phosphatase.

### C. Intracellular Proteins of the Kidney

Proteins located with the lysosomes and intra-cellular space of kidney cells can also used as kidney cell death markers. Examples include enzymes and functional proteins such as glytamyltransferase, N-acetyl-beta-D-glucosaminidase, and lysozyme (Jung Clin Chem 1986).

Tubulointerstitial proteins can also used as kidney cell death markers. Examples include neutrophil gelatinase associated lipocalin (NGAL) and L-type Fatty Acid Binding Protein ( L-FABP). NGAL protein is upregulated predominantly in proximal tubules after stress such as ischemia. Urinary excretion of NGAL reflects proximal tubular cell death. L-FABP protein is upregulated predominantly in proximal tubules after proteinuria. Urinary excretion of L-FABP reflects proximal tubular cell death due to events such as hypoxia or excess proteinuria. The function of the protein is to transport free fatty acids bound to urinary albumin from the urinary lumen to the mitochondria (Kamijo A. J Am Soc Nephrol 10: 106A.1999).

### IV. Detection Methods

Any method of detecting and measuring bikunin and the kidney cell death markerADBP-26 can be used in the present invention. A variety of assays for detecting bikunin andADBP-26 are well known in the art and include, for example, enzyme inhibition assays, antibody stains, latex agglutination, and immunoassays, e.g., radioimmunoassay.

Immunoassays that determine the amount of a protein in a biological sample typically involve the development of antibodies against the protein. The term "antibody" herein is used in the broadest sense and refers to, for example, intact monoclonal antibodies, polyclonal antibodies, multispecific antibodies (e.g., bispecific antibodies), and to antibody fragments that exhibit the desired biological activity (e.g., antigen-binding). The antibody can be of any type or class (e.g., IgG, IgE, IgM, IgD, and IgA) or sub-class (e.g., IgG1, IgG2, IgG3, IgG4, IgA1 and IgA2).

Immunoassays that determine the amount of bikunin in a biological sample typically involve the development of antibodies against bikunin. Polyclonal antibodies produced from rabbits inoculated with purified bikunin are cross-reactive with inter-α-inhibitor and are not specific to the various forms of bikunin and uristatin fragments. Monoclonal antibodies could potentially separate the various forms of bikunin and detect uristatin lacking glycoconjugation. Antibodies for detecting bikunin and ADBP-26 are well known in the art and are not discussed herein, see for example, Pugia et al., 2007, Glycoconj J24;5-15 *and* U.S. Publication 20070020683.

Immunoassays, including radioimmmunoassays and enzyme-linked immunoassays, are useful in the methods of the present invention. A variety of immunoassay formats, including, for example, competitive and non-competitive immunoassay formats, antigen capture assays and two-antibody sandwich assays can be used in the methods of the invention (Self and Cook, Curr. Opin. Biotechnol. 7:60-65 (1996)). In an antigen capture assay, antibody is bound to a solid phase, and sample is added such that the analyte, e.g., bikunin orADBP-26, is bound by the antibody. After unbound proteins are removed by washing, the amount of bound analyte can be quantitated, if desired, using, for example, a radioassay (Harlow and Lane, Antibodies A Laboratory Manual Cold Spring Harbor Laboratory: New York, 1988)). Immunoassays can be performed under conditions of antibody excess, or as antigen competitions, to quantitate the amount of analyte and, thus, determine a level of bikunin and/or ADBP-26.

Enzyme-linked immunosorbent assays (ELISAs) can be used in the present invention. In the case of an enzyme immunoassay, an enzyme is typically conjugated to the second antibody, generally by means of glutaraldehyde or periodate. As will be readily recognized, however, a wide variety of different conjugation techniques exist which are readily available to one skilled in the art. Commonly used enzymes include, for example, horseradish peroxidase, glucose oxidase, β-galactosidase and alkaline phosphatase, amongst others. The substrates to be used with the specific enzymes are generally chosen for the production, upon hydrolysis by the corresponding enzyme, of a detectable color change. It is also possible to employ fluorogenic substrates, for example, which yield a fluorescent product. An enzyme such as horseradish peroxidase (HRP), alkaline phosphatase (AP), β-galactosidase or urease can be linked, for example, to an anti- bikunin antibody or to an anti-ADBP-26 antibody or other anti-kidney cell death marker antibody or to a secondary antibody for use in a method of the invention. A horseradish-peroxidase detection system can be used, for example, with the chromogenic substrate tetramethylbenzidine (TMB), which yields a soluble product in the presence of hydrogen peroxide that is detectable at 450 nm. Other convenient enzyme-linked systems include, for example, the alkaline phosphatase detection system, which can be used, for example, with the chromogenic substrate p-nitrophenyl phosphate to yield a soluble product readily detectable at 405 nm. Similarly, a β-galactosidase detection system can be used with, for example, the chromogenic substrate o-nitrophenyl-β-D-galactopyranoside (ONPG) to yield a soluble product detectable at 410 nm, or a urease detection system can be used with, for example, a substrate such as urea-bromocresol purple (Sigma Immunochemicals, St. Louis, Mo.). Useful enzyme-linked primary and secondary antibodies can be obtained from a number of commercial sources such as Jackson Immuno-Research (West Grove, Pa.).

In certain embodiments, bikunin and/or ADBP-26 can be detected and measured using chemiluminescent detection. For example, in certain embodiments, bikunin specific antibodies are used to capture bikunin present in the biological sample and an antibody specific for the specific antibodies and labeled with an chemiluminescent label is used to detect the bikunin present in the sample. Any chemiluminescent label and detection system can be used in the present methods. Chemiluminescent secondary antibodies can be obtained commercially from various sources such as Amersham. Methods of detecting chemiluminescent secondary antibodies are known in the art and are not discussed herein in detail.

Fluorescent detection also can be useful for detecting bikunin and/or ADBP-26. Useful fluorochromes include, for example, DAPI, fluorescein, lanthanide metals, Hoechst 33258, R-phycocyanin, B-phycoerythrin, R-phycoerythrin, rhodamine, Texas red and lissamine. Fluorescent compounds, can be chemically coupled to antibodies without altering their binding capacity. When activated by illumination with light of a particular wavelength, the fluorochrome-labellcd antibody adsorbs the light energy, inducing a state of excitability in the molecule, followed by emission of the light at a characteristic color visually detectable with a light microscope.

Radioimmunoassays (RIAs) can be useful in certain methods of the invention. Such assays are well known in the art. Radioimmunoassays can be performed, for example, with ¹²⁵I-labeled primary or secondary antibody (Harlow and Lane, supra, 1988).

A signal from a detectable reagent can be analyzed using any methods known in the art. A signal can be measured, for example, using a spectrophotometer to detect color from a chromogenic substrate; a radiation counter to detect radiation, such as a gamma counter for detection of ¹²⁵I; or a fluorometer to detect fluorescence in the presence of light of a certain wavelength. Where an enzyme-linked assay is used, quantitative analysis of the amount of bikunin or ADBP-27 can be performed using a spectrophotometer such as an EMAX Microplate Reader (Molecular Devices; Menlo Park, Calif.) in accordance with the manufacturer's instructions. The assays of the invention can be automated or performed robotically, if desired, and the signal from multiple samples can be detected simultaneously.

The methods of the invention also encompass the use of capillary electrophoresis based immunoassays (CEIA), which can be automated, if desired. Immunoassays also can be used in conjunction with laser-induced fluorescence as described, for example, in Schmalzing and Nashabeh, Electrophoresis 18:2184-93 (1997), and Bao, J. Chromatogr. B. Biomed. Sci. 699:463-80 (1997).

Sandwich enzyme immunoassays also can be useful in certain methods of the invention. In a two-antibody sandwich assay, a first antibody is bound to a solid support, and the antigen is allowed to bind to the first antibody. The amount of the analyte can be quantitated by measuring the amount of a second antibody that binds to it.

Quantitative western blotting also can be used to determine a level of bikunin and/or ADBP-26 in the present methods.

Levels of bikunin and/or ADBP-26 can also be determined using protein microarrays. Methods of producing protein microarrays that can be adapted for detecting levels of protein in a clinical sample are well known in the art.

In certain embodiments, a sample is analyzed by means of a biochip. Biochips generally comprise solid substrates and have a generally planar surface, to which a capture reagent (also called an adsorbent or affinity reagent) is attached. Frequently, the surface of a biochip comprises a plurality of addressable locations, each of which has the capture reagent bound there.

Protein biochips are biochips adapted for the capture of peptides. Many protein biochips are described in the art. These include, for example, protein biochips produced by Ciphergen Biosystems, Inc. (Fremont, CA), Packard BioScience Company (Meriden CT), Zyomyx (Hayward, CA), Phylos (Lexington, MA) and Biacore (Uppsala, Sweden). Examples of such protein biochips are described in the following patents or published patent applications: U.S. Patent No. 6,225,047; PCT International Publication No. WO 99/51773; U.S. Patent No. 6,329,209, PCT International Publication No. WO 00/56934 and U.S. Patent No. 5,242,828.

For use herein, the assay methods can involve capturing bikunin and/or ADBP-26 onto a solid substrate. Typically they will be captured using a biospecific capture reagent such as an antibody and, in particular, an antibody used in an immunoassay. Biospecific capture reagents include those molecules that bind a target analyte with an affinity of, for example, at least 10⁻⁹ M, 10⁻¹⁰ M, 10⁻¹¹ M or 10⁻¹² M. These molecules also can be captured with non-specific methods, such as chromatographic materials.

In certain embodiments of the present invention, bikunin and/or ADBP-26 will be detected by mass spectrometry. Examples of mass spectrometers are time-of-flight, magnetic sector, quadrupole filter, ion trap, ion cyclotron resonance, electrostatic sector analyzer and hybrids of these.

A preferred mass spectrometric technique for use in the invention is "Surface Enhanced Laser Desorption and Ionization" or "SELDI," as described, for example, in U.S. Patents No. 5,719,060 and No. 6,225,047 both to Hutchens and Yip.

This refers to a method of desorption/ionization gas phase ion spectrometry (e.g., laser desorption/ionization mass spectrometry) in which an analyte is captured on the surface of a SELDI probe that engages the probe interface of the mass spectrometer.

In certain embodiments, bikunin and ADBP-26 can also be measured using enzyme inhibition methods and/or kinetic enzymatic assays.

One example of an enzyme inhibition method for measuring bikunin involves the addition to the biological sample of known amounts of trypsin and then measuring the degree to which the trypsin has been inhibited. A substrate is used that is capable of producing a detectable response when it is added to a biological sample and the substrate is cleaved by trypsin to yield detectable byproducts. If trypsin inhibitors are present, the response is reduced, because some of the available substrate is not cleaved. Urinalysis strip methods, such as dipstick methods can be used for the rapid detection of trypsin inhibitors in urine (Pugia et al., Clin Chim Acta, 2004; 341:73-81). Methods of measuring bikunin using enzyme inhibitory assays are well known in the art, see for example, U.S. Patent Nos. 6,955,921, 6,770,764, 7,001,737 and Pugia et al., 2002, Clinical Biochemistry, 32:105-11.

One example of a kinetic enzymatic assay for measuring ADBP-26 involves measuring dipeptidyl peptidase activity using cleavage of peptide substrates, such as, for example, Ala-Pro- p-nitroanilide, and Gly-Pro- p-nitroanilide. Serine peptidase activity can be measured using a chromogenic substrate, e.g., H-Gly-Pro-p-nitroanilide). Enzymatic peptide cleavage assays are known in the art and are thus not discussed herein in detail.

In certain embodiments, bikunin can be measured by measuring the level of expression of the genes encoding for bikunin. Nucleic acid assays are known to those skilled in the art and include, for example, Southern analysis, northern analysis, dot blots, S1 analysis, amplification techniques such as PCR, and *in situ* hybridization.

### V. Kidney Disease Status

Determining kidney disease status generally involves classifying an individual into one of two or more groups based on the results of the diagnostic test. The diagnostic test can be used, for example, to classify between a number of different states, for example between high risk and low risk for developing kidney disease.

In certain embodiments of the present invention, the risk of developing kidney disease will be determined by measuring the levels of bikunin and ADBP-26 and comparing them to a reference amount that is associated with a particular risk level, *i.e.,* correlating the ratio with kidney disease status. In certain embodiments, the ratio of ADBP-26 to bikunin will be determined and the ratio will be compared to a reference amount that is associated with a particular risk level.

In certain embodiments, the present methods can be used to identify whether a subject has bikunin induced inflammatory cell death, non-inflammatory cell death, or bikunin induced inflammation without cell death. A subject having bikunin induced inflammatory cell death will generally have elevated levels of bikunin and elevated levels of ADBP-26. In certain aspects, a bikunin value of greater than 7.8 mg/L in urine is indicative of elevated levels of bikunin; an ADBP-26 value of greater than 50 ng/mL in urine is indicative of elevated levels of ADBP-26; and a bikunin value of greater than 1.4 mg/L in blood is indicative of elevated levels of bikunin. A subject having non-inflammatory cell death will generally have normal levels of bikunin and elevated levels of ADBP-26. A subject having bikunin induced inflammation without kidney cell death will generally have elevated levels of bikunin and normal levels of ADBP-26.

By measuring levels of bikunin and ADBP-26 in a subject at risk for kidney disease, it can be determined whether the a subject is likely to progress to the disease state. The cut-off for ADBP-26 is readily found in the literature and assigned by comparing health patients with very low or no marker in urine to patients with acute renal necrosis. A value higher than the average value of the controls by two to three intra-subject standard deviations is considered positive. An appropriate treatment regimen can be tailored to the subject based upon the levels of bikunin and ADBP-26 in the biological sample obtained from the subject.

In certain embodiments, the present invention provides methods for determining whether a surgical patient is at an increased risk for developing acute renal failure. Acute renal failure (ARF) occurs in approximately 10 % of patients undergoing cardiac bypass surgery. The occurrence of acute renal failure in the post-operative period is associated with a very high mortality such as with patients undergoing bypass surgery found a rate of ARF requiring dialysis of 1.1% with an associated mortality of 63.7% (Chertow et al.,. Am J Med 1998; 4: 343-348). ARF is also associated with post-operative infection, and sepsis (Thakar et al. Kidney Int 2003; 64: 239-246.) Clinical variables and biomarkers can determine the increased risk for ARF in patients undergoing bypass surgery (Loef BG, et al. J Am Soc Nephrol 2005; 16: 195-20). In the highest risk group, ARF occurs in 20-25% of patients. Serum creatinine measurement is currently the most common method to measure acute renal failure.

In certain embodiments of the present invention, the levels of bikunin and ADBP-26 in a subject, *i.e.,* a surgical patient, will be determined in order to determine whether the subject will progress to acute renal failure. By measuring the levels of bikunin and ADBP-26, it can be determined, for example, whether the subject is free from kidney inflammation and kidney cell death or whether the subject has one of bikunin induced inflammatory kidney cell death, non-inflammatory kidney cell death, or bikunin induced inflammation. A subject that is free from kidney inflammation and kidney cell death will have a very low to negligible risk of progressing to acute renal failure while a patient having bikunin induced inflammatory kidney cell death, non-inflammatory kidney cell death, or bikunin induced inflammation will have a higher risk of progressing to acute renal failure. Subjects having bikunin induced inflammation will be more likely to progress to renal failure if the bikunin induced inflammation is paired with kidney cell death. Moreover, by measuring both the levels of bikunin and ADBP-26, an appropriate treatment regimen can be determined. Methods of treating a subject having bikunin induced inflammatory kidney cell death could entail the use of specific therapies for preventing or neutralizing the harmful effects of bikunin whereas such therapies would not be effective in a subject having non-inflammatory kidney cell death.

In certain embodiments of the present invention, measurement of levels of bikunin and ADBP-26 can be paired with measurement of serum creatinine or another biomarker for renal dysfunction in order to determine whether a subject, *e.g.,* a surgical patient, will progress to acute renal failure. For example, subjects who have renal insufficiency (a creatinine level of greater than 1.7 mg/dL in serum) and inflammation induced kidney cell death all progressed to renal failure and are considered to high risk of developing acute renal failure while subjects lacking renal insufficiency but having some inflammation induced kidney cell death are considered to be of lower risk.

The methods of the present invention also include determining a ratio of ADBP-26 to bikunin. An elevated ratio will be indicative of disease. For example, in certain aspects of the present invention, a subject having kidney disease or at a high risk for developing kidney disease will have a ratio of ADBP-26 to bikunin that is at least 5%, 10%, 15%, 20% or 25% greater than a normal control ratio.

A subject having kidney disease or at a high risk for developing kidney disease will have a ratio of ADBP-26 to bikunin of greater than 210 ug ADBP-26 per g bikunin, or even greater than about 340 ug ADBP-26 per g bikunin. In certain embodiments, a ratio of less than 210 ug ADBP-26 per g bikunin will be indicative of a normal control ratio, *i.e.,* it will be indicative of a low risk or normal risk level for developing kidney disease.

### VI. Disease progression/regression

The invention provides methods for determining the course of disease in a subject. Disease course refers to changes in disease status over time, including disease progression (worsening) and disease regression (improvement).

Over time, the levels of bikunin and ADBP-26 change. Whether the levels are increasing or decreasing can indicate the course of disease. Accordingly, this method can involve measuring the levels ofbikunin and ADBP-26 over time , *i.e.,* for at least two different time points, e.g., a first time and a second time, and comparing the change in amounts, if any. The change in the numerical value of the ratio can be indicative of the disease progression or regression.

### VII. Kidney Disease

Inflammation is a hallmark of renal injury and is typically associated with infections. The mechanism of action of bikunin is linked to kidney disease but bikunin is not specific for kidney disease. The kidney cell death marker of the present invention is specific for kidney disease. ADBP-26 has been shown to be a specific indicator of late stage kidney disease. The present inventors have found that bikunin induces kidney cell death and accordingly, a determination of the levels of both bikunin and ADBP-26 can provide essential information regarding the relative risk for developing kidney disease as well as appropriate treatment regimens. The present inventors have also found that the ratio of ADBP-26 to bikunin can provide greater predictive value of a particular status than either alone. In certain embodiments, assessment of one or more additional markers can be combined with the measurement described herein to increase the predictive value of the analysis.

These additional biomarkers can include, for example, kidney markers commonly measured in the urine for assessment of kidney disease but not specific for kidney cell death. Biomarkers that are not specific for kidney cell death generally have a specificity of less than 85%, less than 80%, less than 70% or even less than 60% for kidney disease. Examples generally are biomarkers found in the blood of healthy subjects that are elevated or reduced in the urine after renal injury. Injury to kidney such as in the glomerular or proximal tubular cause changes in filtration and absorption of serum component causing more or less to be spilled into the urine. These biomarkers are excreted into urine from cells else where in the body and not primarily excreted into urine from the kidney cell. Nor are the changes in urinary levels of these biomarkers primarily due to kidney cell death. They are often due to other conditions such as infection, auto-immune disease, cancer, cardiovascular disease, and others. As such they do not meet the definition of specific markers of kidney cell death and are urinary markers dependent on kidney filtration and reabsorption.

Urinary markers dependent on kidney filtration and reabsorption include small molecules, low molecular weight proteins, immuno reactive components, high molecular weight proteins and urine sediment. Examples of small molecules are urea, glucose, amino acids, phosphate, prostaglandins, creatinine and markers of urine concentrations such as specific gravity, salts or water. Examples of low molecular weight proteins ~<30 kDa include alpha-1-microglobulin, retinol binding protein, glutathione-S-transferases, adiponectin, and beta- 2-macroglobuin, calbindin-D, cysteine-rich protein 6, endothelial/epithial growth factors and others associated with the serum proteome. Examples of immuno reactive components immunoglobuin and complements include IgA, IgG, gG, IgE, IgD, IgM, renal tubule brush border antigens, and kappa/lamda light chains and cytokine/chemokines like the interleukins (IL-1, IL-6, IL-18). Examples of high molecular weight proteins are defined as ~>45 kDa and include examples such as alpha-1-acid glycoprotein, prealbumin, modified albumin, albumin, transferrin, alpha-1-lipoprotein, alpha-1-antitrypsin matrix metalloproteinases (MMPs), alpha-1- fetoprotein, Tamm Horsfall protein and alpha-1- glycoprotein. Examples of urine sediment include cell, cast and crystals.

The methods provided herein can also be used in combination with any other tests that will aid in the diagnosis of a disease, determination of progression of a disease, or determination of efficacy of treatment, e.g., renal imaging.

As used herein the term kidney disease is meant to include any disease affecting the kidneys and specifically, those diseases that are associated with inflammation of the kidney. Exemplary diseases include, for example, nephritis, nephrotoxicity, renal failure, diabetic nephropathy, kidney cancer, glomerulonephritis. Kidney disease includes kidney disease associated with other conditions, including, for example, hypertension, diabetes, and autoimmune disease, such as, for example, lupus.

### VIII. Kits for detection of ADBP-26 to bikunin ratios

The present invention provides kits for determining kidney diseases status. The kit comprises a means for measuring ADBP-26 and bikunin in biological sample.

In addition, the kits can include instructional materials containing directions (i.e., protocols) for the practice of the methods of this invention. While the instructional materials typically comprise written or printed materials they are not limited to such. Any medium capable of storing such instructions and communicating them to an end user is contemplated by this invention. Such media include, but are not limited to electronic storage media (e.g., magnetic discs, tapes, cartridges, chips, and the like), optical media (e.g., CD ROM), and the like. Such media can include addresses to internet sites that provide such instructional materials.

The kit can also comprise, for example, a means for obtaining a biological sample from an individual. Means for obtaining biological samples from individuals are well known in the art, e.g., catheters, syringes, and the like, and are not discussed herein in detail.

### IX. Examples

### Example 1 - Correlation of bikunin and ADBP-26

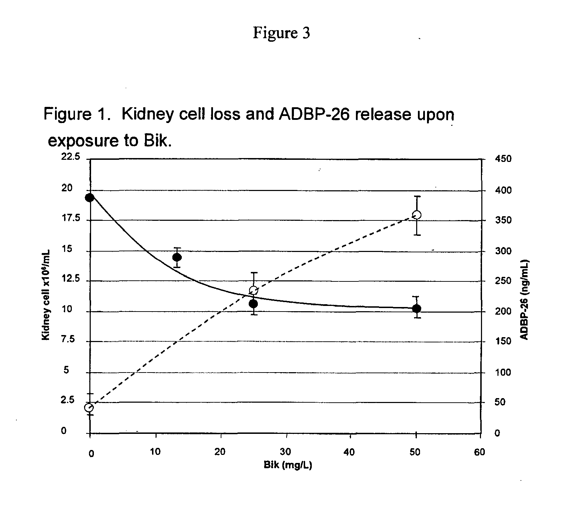

The impact of Bik (12.5 to 500 mg/L) on normal kidney cells was measured by the exposure of synchronized cultured kidney cells to Bik. Exposures and analysis were preformed on three separate trials. Cells were harvested after 24 hours from exposure to Bik. Cell counts always decreased with increased Bik concentration indicating increased cell death as shown in Figure 3 (closed circles). There was an increased change in the amount of ADBP-26 indicating an increase in cell kidney cell death (open circles). Normal kidney cell did not contain Bik at concentrations found during inflammation. The results support the finding that excess inflammation causes cell death and release of ADBP-26. Bik was also protecting the cells from immune mediated apoptosis and reduced cell proliferation due to inflammatory response. Bik inhibited serine protease involved in cell signaling and in adsorption of protein by the kidney.

### Example 2 - Ratio of ADBP-26 to bikunin in hospital patients.

A set of hospital patient samples were collected from patients with and without kidney disease. Patients were diagnosed with renal insuffiency when consistently presenting with proteinuria > 1 g/day. Urine samples were tested for ADBP-26 and Bik to demonstrate the relationships in kidney disease. Individually, Bik and ADBP-26 correlate to presence of kidney disease. The relationships of ADBP-26 and Bik were tested as well. In this example a ratio of ADBP-26 and Bik was used. A ratio is only one example of a metric using both results. The following data supports a correlation of ADBP-26/Bik ratio to kidney disease and that ratio further increased the correlation to kidney disease beyond the two markers individually. Thirty four normal controls and four kidney patients were tested. The urinary ADBP-26 values for normals were all less than 39 ng/mL (this threshold is the average value plus 3 SD). Using >39 ng/mL as the threshold for a positive ADBP -26 result, all of the normals were negative and only one of the four patient samples was positive. The first patient had a high ADBP -26 measurement of 86 ng/mL. The urinary Bik reference range for a normal result is < 7.8 mg/L based on literature. Using >=7.8 mg/L as the threshold for a positive Bik result, eight of the normal controls were positive for Bik and all of the four patient samples were positive. Using the ADBP-26 to Bik ratio, all four patients were positive with a ratio of 21.0 mg/g and all of normal control patients were negative with a ratio < 21.1 mg/g. The urinary ratio of ADBP-26/ Bik clarifies the population at risk for kidney failure. Most patients are below 210 ug ADBP-26/ g Bik, and then there is a large change in the ratio to above 340 with four patients having high risk of kidney damage (or vascular damage).

**Table 1. Ratio for ADBP-26 and Bik**

| Number | | Average ug ADBP-26/ g Bik | SD ug ADBP-26/ g Bik | Max ug ADBP-26/ g Bik | Min ug ADBP-26/ g Bik |
|---|---|---|---|---|---|
| 34 | Normals | 38 | 21 | 123 | 0.3 |
| 4 | Abnormals | 507 | 178 | 736 | 341 |

Overall data supports urinary measurement of ADBP-26 and Bik is better for risk stratification of kidney patients than either Bik or ADBP-26 use alone. The risk level is easily expressed as the ratio of ADBP-26/Bik. In this example, patients with a higher ratio are at the highest risk.

### Example 3 - Levels of bikunin and ADBP-26 in surgical patients

A clinical study was done for a group of patients undergoing cardiac bypass surgery to determine the time course of ADBP-26 and bikunin appearance in the urine in patients at risk for acute renal failure (as defined by a 30% rise in serum creatinine) following cardiac bypass surgery and measured for at least 3 days during the course of the surgery. Patients were risk stratified according to the Cleveland Clinic Foundation Pre-operative Risk Score (Loef) to allow a good percentage of those going on to failure. The ADBP-26 and bikunin are compared to the serum creatinine for the following patients who when onto ARF and those who retained normal kidney function.

All samples were examined for the biomarkers and patients were considered of low risk if the urine Bik, urine ADBP-26 and serum creatinine was normal. 7.8 mg/L was used as the cutoff for Bik; 50 ng/mL was used as the cutoff for ADBP-26 and 1.7 was used as the cutoff for serum creatinine. Patients were considered of normal range if they had inflammation without induced kidney cell death or renal insufficiency (or a Bik > 7.8 mg/L, ADBP-26 <50 ng/mL and serum creatinine <= 1.7 mg/dL). Patients were considered of low risk but still in the normal range if they had inflammation induced kidney cell death without renal insufficiency (or a Bik > 7.8 mg/L, ADBP-26 > 50 ng/mL and serum creatinine <= 1.7 mg/dL). In these cases, the patient status returned to normal during the three days or was noted on day three only. Patients were considered of highly likely for renal failure if they had renal insufficiency (creatinine > 1.7 mg/dL) due to inflammation induced kidney cell death (Bik > 7.8 mg/L, ADBP-26 > 50 ng/mL) or due to had inflammation without kidney cell death (Bik > 7.8 mg/L, ADBP-26 <50 ng/mL). Patient with non-inflammatory kidney cell death were of medium risk to have renal insufficiency or progress to failure (Bik< 7.8 mg/L, ADBP-26 > 50 ng/mL). As can be seen by the table below all surgery patients who have progressed to renal failure are indicated by the diagnostic relationships indicating medium or high risk.

**Table 2. Assessment of Surgical Patients for Renal Failure**

| Diagnosis | Surgery patients who do not have renal failure | Surgery patients who have renal failure |
|---|---|---|
| Normal low risk | 41 | 0 |
| Anti-inflammatory response with out kidney cell death or renal insufficiency | 30 | 0 |
| Inflammation induced kidney cell death without renal insufficiency | 29 | 0 |
| Non-inflammatory kidney cell death and without renal insufficiency | 6 | 0 |
| Renal insufficiency due non-inflammatory kidney cell death | 6 | 12 |
| Renal insufficiency due to inflammation induced kidney cell death | 0 | 23 |
| Renal insufficiency due to inflammation without kidney cell death | 0 | 15 |

As can be seen from the table, about half (23) of the patients with inflammation induced kidney cell death (23+29) progressed to renal failure during the operation. Where as a quarter of patients (15) with inflammation but no kidney cell death (45) progressed to renal failure during the operation. This means that as kidney cell death occurs failure is more likely.

At the same time about half of patients (12) with non-inflammation kidney cell death (24) progressed to renal failure. These patients would be treated differently as other causes of kidney cell death are occurring. A Bik preventing therapy would not work.

Using only a marker of kidney cell death would miss-identify therapies preventing Bik induced kidney cell death for patient (12 +15) and controls (29+6+6). Using just Bik as marker of kidney inflammation would miss-identify therapies preventing Bik induced kidney cell death for no patients but would misidentify many controls (29+30). Overall no negatives but more false positives than the kidney cell death marker. Using both would miss-identify therapies in patient (15) and controls (29). Using both markers gave less false positives and less false negatives than when using a kidney cell death marker alone.

## Claims

1. A method for identifying whether a subject has kidney disease or is at an increased risk for developing kidney disease, the method comprising determining the level of bikunin and the level of adenosine deaminase binding protein (ADBP-26) in a urine sample obtained from the subject and determining that the subject has kidney disease or is at an increased risk for developing kidney disease based on an elevated ratio of ADBP-26 to bikunin as compared to a normal control ratio determined from individuals without kidney disease.

2. The method of claim 1 further comprising determining if the subject has bikunin induced inflammatory kidney cell death based on an elevated level of bikunin and an elevated level of ADBP-26.

3. The method of claim 1 further comprising determining if the subject has non-inflammatory kidney cell death based on a normal level ofbikunin and an elevated level of ADBP-26.

4. The method of claim 1 further comprising determining if the subject has bikunin induced inflammation without kidney cell death based on an elevated level of bikunin and a normal level of ADBP-26.

5. The method of claim 1 wherein a ratio of greater than 210 ug, preferably of greater than 340 ug ADBP-26 per g bikunin identifies the subject as suffering from kidney disease or having an increased risk for developing kidney disease.

6. The method of claim 1 wherein the method is for identifying a subject at an increased risk for developing renal failure.

7. The method of claim 1 further comprising the step of determining the level of a biomarker that can be measured in the urine for assessment of kidney disease but that is not specific for kidney cell death.

8. The method of claim 1 further comprising the step of determining the level of a biomarker for renal dysfunction.

9. A method for measuring the course of kidney disease comprising
determining at a first time point, the level of bikunin and the level of ADBP-26 in a urine sample obtained from the subject, and determining the ratio of ADBP-26 to bikunin;
determining at a second time point, the level of bikunin and the level of ADBP-26 in a urine sample obtained from the subject, and determining the ratio of ADBP-26 to bikunin;
and comparing the first measurement and the second measurement; wherein a ratio trending to normal indicates kidney disease regression, and
wherein and a ratio trending to an elevated ratio indicates kidney disease progression.

10. A kit comprising a means for measuring adenosine deaminase binding protein (ADBP-26) and bikunin in a biological sample; and optionally instructions for use and interpretation of the kit results.

## Patentansprüche

1. Verfahren zum Feststellen, ob ein Patient an einer Nierenkrankheit leidet oder ob bei dem Patienten ein erhöhtes Risiko des Auftretens einer Nierenkrankheit besteht, wobei man bei dem Verfahren in einer von dem Patienten erhaltenen Urinprobe die Konzentration an Bikunin und die Konzentration an Adenosindeaminase-bindendem Protein (ADBP-26) bestimmt und anhand eines im Vergleich zu einem bei Individuen ohne Nierenkrankheit bestimmten Vergleichsverhältnisses erhöhten Verhältnisses von ADBP-26 zu Bikunin bestimmt, dass der Patient an einer Nierenkrankheit leidet oder dass bei dem Patienten ein erhöhtes Risiko des Auftretens einer Nierenkrankheit besteht.

2. Verfahren nach Anspruch 1, bei dem man weiterhin anhand einer erhöhten Konzentration von Bikunin und einer erhöhten Konzentration von ADBP-26 bestimmt, ob der Patient an Bikunininduziertem entzündlichen Nierenzellentod leidet.

3. Verfahren nach Anspruch 1, bei dem man weiterhin anhand einer normalen Konzentration an Bikunin und einer erhöhten Konzentration an ADBP-26 bestimmt, ob der Patient an nichtentzündlichem Nierenzellentod leidet.

4. Verfahren nach Anspruch 1, bei dem man weiterhin anhand einer erhöhten Konzentration an Bikunin und einer normalen Konzentration an ADBP-26 bestimmt, ob der Patient an Bikunininduzierter Entzündung ohne Nierenzellentod leidet.

5. Verfahren nach Anspruch 1, wobei ein Verhältnis von über 210 µg, vorzugweise über 340 µg ADBP-26 pro g Bikunin besagt, dass der Patient an einer Nierenkrankheit leidet oder dass bei dem Patienten ein erhöhtes Risiko des Auftretens einer Nierenkrankheit besteht.

6. Verfahren nach Anspruch 1, wobei das Verfahren zum Identifizieren eines Patienten ist, bei dem ein erhöhtes Risiko des Auftretens von Nierenversagen besteht.

7. Verfahren nach Anspruch 1, welches weiterhin den Schritt der Bestimmung der Konzentration eines im Urin messbaren biologischen Markers, der jedoch nicht spezifisch für Nierenzellentod ist, zur Untersuchung auf eine Nierenkrankheit umfasst.

8. Verfahren nach Anspruch 1, welches weiterhin den Schritt der Bestimmung der Konzentration eines biologischen Markers für Nierendysfunktion umfasst.

9. Verfahren zum Messen des Verlaufs einer Nierenkrankheit, bei dem man zu einem ersten Zeitpunkt die Konzentration an Bikunin und die Konzentration an ADBP-26 in einer von dem Patienten erhaltenen Urinprobe bestimmt und das Verhältnis von ADBP-26 zu Bikunin bestimmt,
zu einem zweiten Zeitpunkt die Konzentration an Bikunin und die Konzentration an ADBP-26 in einer von dem Patienten erhaltenen Urinprobe bestimmt und das Verhältnis von ADBP-26 zu Bikunin bestimmt,
und die erste Messung mit der zweiten Messung vergleicht, wobei ein sich zu Normalwerten verschiebendes Verhältnis auf eine Regression der Nierenkrankheit hindeutet, und
wobei ein sich zu einem erhöhten Verhältnis verschiebendes Verhältnis auf eine Progression der Nierenkrankheit hindeutet.

10. Kit, umfassend ein Mittel zum Messen von Adenosindeaminase-bindendem Protein (ADBP-26) und Bikunin in einer biologischen Probe und gegebenenfalls Anweisungen zum Gebrauch und zur Interpretation der Ergebnisse des Kits.

## Revendications

1. Procédé pour reconnaître si un sujet a une maladie rénale ou présente un risque accru d'en avoir une, le procédé comprenant la détermination du taux de bikunine et du taux de protéine de fixation de l'adénosine désaminase (ADBP-26) dans un échantillon d'urine du sujet et la détermination que le sujet a une maladie rénale ou présente un risque accru d'en avoir une sur la base d'un rapport élevé de L'ADBP-26 à la bikunine par rapport à un rapport témoin normal déterminé chez des individus sans maladie rénale.

2. Procédé suivant la revendication 1, comprenant en outre la détermination du point de savoir si le sujet a une mortalité inflammatoire de cellule rénale induite par la bikunine sur la base d'un taux élevé de bikunine ou d'un taux élevé d'ADBP-26;

3. Procédé suivant la revendication 1, comprenant en outre la détermination du point de savoir si le sujet a une mortalité non inflammatoire de cellule rénale sur la base d'un taux normal de bikunine et d'un taux élevé d'ADBP-26.

4. Procédé suivant la revendication 1, comprenant en outre la détermination du point de savoir si le sujet a une inflammation induite par la bikunine sans mortalité de cellule rénale sur la base d'un taux élevé de bikunine et d'un taux normal d'ADBP-26.

5. Procédé suivant la revendication 1, dans lequel un rapport supérieur 210 ug, de préférence supérieur à 340 ug d'ADBP-26 par gramme de bikunine montre que le sujet souffre d'une maladie rénale ou présente un risque accru d'en avoir une.

6. Procédé suivant la revendication 1, dans lequel le procédé est destiné à reconnaître qu'un sujet a un risque accru d'avoir une défaillance rénale.

7. Procédé suivant la revendication 1, comprenant en outre le stade de détermination du taux d'un biomarqueur, qui peut être mesuré dans l'urine pour établir qu'il y a une maladie rénale mais qui n'est pas spécifique à une mortalité de cellule rénale.

8. Procédé suivant la revendication 1, comprenant en outre le stade de détermination du taux d'un biomarqueur pour un disfonctionnement rénal.

9. Procédé de mesure de l'évolution d'une maladie rénale, comprenant
la détermination en un premier point dans le temps du taux de bikunine et du taux d'ADBP-26 dans un échantillon d'urine d'un sujet et la détermination du rapport de l'ADBP-26 à la bikunine :
la détermination en un deuxième point dans le temps du taux de bikunine et du taux d'ADBP-26 dans un échantillon d'urine du sujet et la détermination du rapport de l'ADBP-26 à la bikunine ;
et la comparaison de la première mesure et de la deuxième mesure, un rapport tendant vers la normale indiquant une régression de la maladie rénale, et
dans lequel un rapport tendant vers un rapport élevé indiquant une progression de la maladie rénale.

10. Trousse comprenant un moyen de mesure de la protéine de fixation de l'adénosine désaminase (ADBP-26) et de la bikunine dans un échantillon biologique ; et éventuellement des instructions d'utilisation et d'interprétation des résultats de la trousse.
